# EUROPEAN PATENT APPLICATION

(11) **EP 2 484 779 A1**
(43) Date of publication of application: **08.08.2012**
(21) Application number: 11153273.5
(22) Date of filing: 03.02.2011
(51) Int. Cl.: C12Q 1/68

(54) **Device and method for analysis of kidney failure**

(71) Applicant: Medizinische Hochschule Hannover, 30625 Hannover (DE)
(72) Inventor: Thum, Thomas Prof. Dr., 30659 Hannover (DE); Lorenzen, Johan Dr., 30175 Hannover (DE); Kielstein, Jan Dr., 39112 Magdeburg (DE)
(74) Representative: Taruttis, Stefan Georg

(57) **Abstract**

The invention provides a method for analysis of the status of kidney function in a sample of body fluid obtained from a patient who has not been diagnosed with acute kidney injury (AKI, also referred to as acute kidney failure) or in a sample of body fluid obtained from a patient who has been diagnosed with AKI, which analysis is suitable for evaluating the prospects for long-term survival, which method for analysis comprises or consists of determining the concentration of the microRNA-210

SEQUENCE LISTING

<110>  Medizinische Hochschule Hannover

<120>  Device and method for analysis of kidney failure

<130>  M1038EP

<160>  4

<170>  PatentIn version 3.5

<210>  1
<211>  22
<212>  RNA
<213>  Homo sapiens


<220>
<221>  misc_RNA
<222>  (1)..(22)

<400>  1
cugugcgugu gacagcggcu ga                                                22


<210>  2
<211>  22
<212>  RNA
<213>  Homo sapiens


<220>
<221>  misc_RNA
<222>  (1)..(22)

<400>  2
aaaagcuggg uugagagggc ga                                                22


<210>  3
<211>  22
<212>  RNA
<213>  Homo sapiens


<220>
<221>  misc_RNA
<222>  (1)..(22)

<400>  3
uagcagcacg uaaauauugg cg                                                22


<210>  4
<211>  24
<212>  RNA
<213>  Caenorhabditis elegans


<220>
<221>  misc_RNA
<222>  (1)..(24)

<400>  4
uacccguaau cuucauaauc cgag                                              24

## Description

The present invention relates to an analytical method, and to a kit of parts, which is adapted to the analytical method, for analyzing a patient sample in order to determine the prospects of a patient to develop acute kidney injury (AKI), and to determine the prospects of patients suffering from AKI for long term survival, e.g. for 28 days from the diagnosis of AKI. The invention provides for an analyte, and for the use of the analyte, which in respect to AKI is essentially independent from a diagnosed sepsis or shock. The concentration of the analyte of the invention, preferably in combination with the concentration of one or both of two additional analytes in a sample of body fluid, e.g. in blood serum obtained from patients, especially from patients suffering from AKI, is a marker of the propensity to develop AKI, and for survival, e.g. of 28-day survival, including renal recovery.

### State of the art

Juan et al. in Urology 835-841 (2010) describe the identification of microRNAs which are characteristic for kidney cancer in comparison to microRNAs from normal kidney.

Mitchell et al., PNAS 10513-10518 (2008) describe circulating microRNAs as markers for cancer, especially of miR-141 as a characteristic for prostate cancer.

Ali et al., J. Am. Soc. Nephrol. 1292-1298 (2007) describe the analysis of creatinine values related to acute kidney injury and acute-on-chronic renal failure.

Bagger et al., Pediatr. Nephrol. 1655-1658 (2007) describe serum creatinine levels and urine output as measures that reflect renal function.

US 2010/0173288 Al describes that generally disease states can be analysed by measurement of all detectable microRNAs in blood serum for a patient, and describe a kit and biochip, respectively, containing essentially all mature microRNAs known from human serum.

### Objective of the invention

It is an object of the invention to provide for an analytical method, and for a kit of parts suitable for performing the method, suitable for evaluating the risk of patients to develop AKI, and suitable for evaluating the risk of mortality, and the prospects of long-term survival and renal recovery, respectively, in AKI patients.

### General description of the invention

The invention achieves the above-mentioned objective by providing a method for analysis of the status of kidney function in a sample of body fluid obtained from a patient who has not been diagnosed with acute kidney injury (AKI, also referred to as acute kidney failure) or in a sample of body fluid obtained from a patient who has been diagnosed with AKI, which analysis is suitable for evaluating the prospects for long-term survival, e.g. for 28 days from the diagnosis of AKI, and/or for renal recovery in an AKI patient, which method for analysis comprises or consists of determining the concentration of the microRNA-210 (miR-210) comprising or consisting of the nucleic acid sequence SEQ ID NO 1: CUGUGCGUGUGACAGCGGCUGA, preferably in combination with determining the concentration of one or both of miR-320 comprising or consisting of the nucleic acid sequence SEQ ID NO 2: AAAAGCUGGGUUGAGAGGGCGA and miR-16 comprising or consisting of the nucleic acid sequence SEQ ID NO 3: UAGCAGCACGUAAAUAUUGGCG in a sample, preferably in a serum sample obtained from the patient, which patient can be diagnosed to suffer from AKI, e.g. by analysis of creatinine levels.

The invention is based on the finding that the concentration of the analyte miR-210, preferably in combination with one or both of miR-320 and miR-16, is significantly correlated with the propensity of a patient to initially develop AKI, and with the prospects of survival, e.g. for 28 days, and for renal recovery of AKI patients. In greater detail, it has been found that a serum concentration of miR-210 that is elevated in comparison to median serum levels is indicative of a propensity of a person to develop AKI, and of a reduced prospect for long-term survival of AKI patients, but is generally indicative of an increased mortality. The median serum level can e.g. be the concentration of the respective microRNA found in healthy persons, especially of miR-210s.

Concentrations of miR-320 which are decreased below median serum concentrations of healthy persons are also indicative of an increased propensity to initially develop AKI, indicative of an increased mortality, and indicative of a reduced prospect of long-term survival in AKI patients, and concentrations of miR-16 decreased below a median concentration in healthy persons are also indicative of an increased mortality.

In a first embodiment, the analytical method for determining the concentration of miR-210, optionally in combination with determining the concentration of miR-320 and/or of miR-16, allows the evaluation of the risk of a patient to develop AKI, and/or of the propensity of a patient to suffer from or develop AKI, as the concentration of the analytes of the invention is a predictive indicator for AKI, which indicator is independent from other known indicators for AKI, e.g. from diagnosis of sepsis or shock, and essentially independent from diagnosis of creatinine levels and/or from urine output.

Generally, the method for analysis can be performed by detecting the concentration of the microRNAs which are used as the analyte, i.e. miR-210, preferably additionally miR-320 and/or miR-16 in a sample of body fluid for determining the concentration of the analyte miRNA. The step of determining the concentration of the analyte in a first embodiment generally comprises the step of contacting a sample of body fluid with a nucleic acid sequence which comprises or consists of a section that is reverse complementary to the analyte microRNA (miR), and detecting the concentration or number of hybrids formed by the reverse complementary nucleic acid section with sample components. For detecting the concentration or number of analyte-specific hybrids, it is preferred that the nucleic acid sequence comprising or consisting of a nucleic acid section which is reverse complementary to the analyte miR is immobilized on a substrate, e.g. the nucleic acid sequence can be contained in a panel of nucleic acid sequences which are immobilized and spaced apart on a carrier substrate, such as panels or nucleic acid arrays in the form of a chip.

In a further embodiment, the step of determining the concentration of the one or more analyte miR can be performed by reverse transcription and PCR (RT-PCR) using an oligonucleotide primer which is specific for the analyte miR, followed by reverse transcription and amplification of at least a section of the analyte miR by PCR. For detection, hybridizing probes which are labelled with a chromophor and a spaced apart quencher, the probe having an analyte-specific nucleic acid sequence can be used, e.g. as known for the TaqMan PCR.

It is generally preferred that the analytical method is performed using an internal control for the reactions of determining the concentration of the analyte miR, e.g. by adding a known miR as a control to the sample and analysing the concentration of the added control miR by parallel detection with specificity for the control miR. The miR which is added to the sample as an internal control for the analytical method can be a known concentration or copy number of the analyte miR, e.g. a known copy number of miR-210, miR-320 and/or of miR-16, and/or an miR of human or non-human origin, e.g. a synthetic or bacterial microRNA. Preferably, the added internal control miR is produced by bacterial transcription from a DNA sequence encoding the miR, or is produced by enzymatic synthesis, e.g. by PCR, or by chemical synthesis.

The specific amplification of the added internal control miRNA is preferably used for normalization of the determined initial or original serum concentration or copy number of analyte miRNA, e.g. miR-210, miR-320 and/or of miR-16. The initial serum concentration of analyte miRNA was determined by applying a standard curve for the relation of measured amplification of added analyte miRNA of known concentration to the measured number of amplification products obtained in RT-PCR for analyte miRNA. In detail, a standard curve was generated by adding a known copy number (concentration) of analyte miRNA, e.g. of miR-16, miR-210 and miR-320, e.g. obtainable from Ambion Co. at 0.2 fmol/µL analyte miRNA, performing RT-PCR with primers specific for analyte miRNA, and measuring the copy number of specific amplification products. In addition to adding analyte miRNA of known concentration, internal control miRNA, e.g. miR-54 of C. elegans of known copy number and primers specific for internal control miRNA were added. The original serum concentration of analyte miRNA could be calculated by applying the relation of the copy number of analyte-specific amplification products to the original concentration of analyte miRNA, which relation is determined in the standard curve. Normalization could be obtained by dividing the determined concentration of original concentration of analyte miRNA by the concentration of added internal control miRNA.

Accordingly, the method for analysis preferably comprises the calculation of the concentration of the analyte miRNA by means of a standard curve that contains the pre-determined correlation between the concentration of detected analyte miRNA-specific amplification products obtained in RT-PCR to an original (initially known) concentration of analyte miRNA in serum.

In order to account for variability of RNA isolation from serum, the method preferably contains the step of adding a known concentration of a known internal control miRNA to the serum, isolating RNA from the serum, carrying out RT-PCR on the RNA with specific primer pairs added for each of the analyte miRNA and for the control miRNA, respectively, and in addition to calculating the original concentration of analyte miRNA by means of the standard curve, the method also contains the step of calculating the original concentration of added control miRNA by means of a pre-determined standard curve which contains the pre-determined correlation of the detected concentration of amplification products of the control miRNA to the original concentration of control miRNA, and forming the quotient of the calculated original concentration of analyte miRNA and the calculated concentration of control miRNA. Accordingly, it is generally preferred to determine the concentration of analyte miRNA in the serum sample as a quotient to a control miRNA. For example, if control miR-54 of C. elegans was added to 5 fmol/µL serum, for miR-210 as the analyte miRNA, the quotient of (calculated initial concentration of miR-210)/(calculated concentration of control miR-54 (C. elegans)) above 4.00098 x 10⁻⁵ in AKI patients were found to be indicative of a worse prognosis for survival than a lower quotient. The control miRNA preferably is an added miRNA, preferably of a non-human nucleotide sequence, or an miRNA which is determined or known to be present at constant concentrations in human serum.

Preferably, the method of analysis, as well as the determination of the standard curve for analyte miRNA and control miRNA, respectively, are carried out using at least 2 different dilutions of the isolated RNA, e.g. 2 to 6 or 3 to 4 dilutions, each e.g. diluted by a factor of 5 to a factor of 10.

In a further embodiment, the invention relates to the use of nucleic acid sequences containing or consisting of a section that comprises or consists of the nucleic acid sequence of at least one of the analyte microRNAs, and/or to the use of nucleic acid sequences containing or consisting of a section that comprises or consists of a nucleic acid sequence is reverse complementary to at least one of the analyte microRNAs, e.g. to nucleic acid sequences containing or consisting of a nucleotide sequence of miR-210, and/or to nucleic acid sequences containing or consisting of a nucleotide sequence which is reverse complementary to miR-210, optionally additionally to nucleic acid sequences containing or consisting of miR-320, and/or miR-16 and/or nucleic acid sequences containing or consisting of a section that is reverse complementary to miR-320, and/or miR-16, as an indicator substance which is specific for kidney function, especially for use as an indicator substance that is specific for the propensity to suffer from or to develop AKI, and/or as an indicator substance, the concentration of which in a sample of body fluid is indicative of the propensity for an increased mortality for AKI patients.

### Detailed description of the invention

The invention is now described in greater detail by way of examples with reference to the figures, wherein
- Figure 1 shows the concentration of miR-16 in patients with acute kidney injury (AKI), and in healthy control individuals,
- Figure 2 shows the levels of miR- 320 in AKI patients and in healthy control individuals (CTL),
- Figure 3 shows the concentration of miR-210 in AKI patients and in healthy individuals (CTL),
- Figure 4 shows the concentration of miR-16 in AKI patients grouped for survivors and non-survivors,
- Figure 5 shows the concentration of miR-320 grouped for survivors and non-survivors,
- Figure 6 shows the concentration of miR-210 in AKI patients, grouped for survivors and non-survivors, and
- Figure 7 shows the fraction of surviving patients grouped for concentrations of miR-210 below a median concentration, and concentrations above a median concentration, respectively, over the time scale.

In the analysis of 77 AKI patients and of 30 healthy age-matched individuals which served as control (CTL), micro-RNAs were quantitatively measured by RT-PCR from serum samples. It was found that in AKI patients, concentrations of miR-210 were higher (p < 0.0001), while concentrations of miR-16 (p < 0.0001) and miR-320 (p < 0.0001) were lower in comparison to healthy individuals. This result shows that a concentration of miR-210 higher than the median concentration in a healthy individual is indicative of AKI or of a significant propensity to suffer from or to develop AKI, whereas concentrations of miR-16 and/or of miR-320 below the median concentration of healthy individuals is also indicative of AKI, or of a significant propensity to suffer from or to develop AKI.

Multivariate Cox regression and Kaplan-Meier curve analyses revealed that the concentration of miR-210 can be used as an independent analyte indicative of the propensity to suffer from or to develop AKI, or for the presence of AKI, as an increased concentration of miR-210 above the median concentration of this analyte in healthy individuals in an independent indicator which is correlated with a reduced 28 - day survival (p = 0.05 and p = 0.03, respectively). Detailed demographic, clinical and laboratory characteristics of a total of 77 patients are shown in Table 1, wherein

APACHE II = acute physiology and chronic health evaluation II, BMI = body mass index, CRP = C-reactive protein, eGFR = estimated glomerular filtration rate; ICU = intensive care unit; MAP = mean arterial blood pressure; n = number of patients; RRT = renal replacement therapy; SOFA = sequential organ failure assessment:

**Table 1: Demographic, clinical and laboratory characteristics of patients**

| | Total | survivors | non-survivors | p-value |
|---|---|---|---|---|
| Number of patients | 77 | 49 | 28 | 0.7 |
| Male (n; %) | 40 (52) | 26 | 14 | |
| Female (n; %) | 37 (48) | 23 | 14 | |
| Discipline of ICU admission | | | | 0.9 |
| Medicine (n; %) | 29 (38) | 19 (40) | 10 (36) | |
| General surgery (n; %) | 20 (26) | 13 (27) | 7(25) | |
| Cardiac surgery (n; %) | 28 (36) | 17 (35) | 11 (39) | |
| Age (years) | 49 (39-60) | 49 (40-61) | 50 (38-60) | 0.8 |
| BMI (kg/m²) | 25 (22-28) | 25 (22-28) | 25 (22-28) | 0.6 |
| Indication for RRT | | | | |
| eGFR loss >30 % | 68 | 44 | 24 | 0.4 |
| Oliguria/anuria | 48 | 30 | 18 | 0.9 |
| Metabolic acidosis | 7 | 1 | 6 | 0.005* |
| Hyperkalemia | 6 | 2 | 4 | 0.1 |
| SOFA score | 13 (11-16) | 12 (10-15) | 14 (13-18) | 0.05* |
| | | | | |
| RIFLE class | | | | 0.2 |
| Risk (n; %) | 5 (6) | 2 (4) | 3 (11) | |
| Injury (n;%) | 21(27) | 16 (33) | 5 (18) | |
| Failure (n; %) | 50 (65) | 30 (63) | 20 (71) | |
| APACHE II score | 29 (25-34) | 28 (23-34) | 34 (29-38) | 0.4 |
| CRP (mg/L) | 128 (47-202) | 96 (48-205) | 141 (56-198) | 0.5 |
| MAP (mmHg) | 82 (71-94) | 84 (70-97) | 78 (73-86) | 0.3 |
| Heart rate (bpm) | 99 (88-109) | 92 (83-104) | 100 (96-117) | 0.6 |
| miR-16 | 1.9x10⁻⁴ | 1.9x10⁻⁴ | 2.2x10⁻⁴ | 0.9 |
| | (9.7x10⁻⁵- | (9.4x10⁻⁵- | (1.2x10⁻⁴- | |
| | 4.9x10⁻⁴) | 4x10⁻⁴) | 1.2x10⁻³) | |
| miR-210 | 4x10⁻⁵ | 3x10⁻⁵ | 7x10⁻⁵ | 0.02* |
| | (1.4x10⁻⁵- | (1x10⁻⁵ | (2.8x10⁻⁵- | |
| | 1x10⁻⁴) | 7.6x10⁻⁵) | 1.2x10⁻⁴) | |
| miR-320 | 1x10⁻⁴ | 1x10⁻⁴ | 1.7x10⁻⁴ | 0.03* |
| | (5x10⁻⁵- | (4x10⁻⁵- | (8.3x10⁻⁵- | |
| | 3.4x10⁻⁴) | 2.8x10⁻⁴) | 4.1x10⁻⁴) | |

These data show that no association between the microRNAs analysed and the APACHE II score or the SOFA score can be found. Further, no significant differences in concentrations of the microRNAs indicated in patients with or without sepsis (miR-16: p=0.3, miR-210: p=0.4; miR-320: p=0.6), surgery (miR-16: p=0.4; miR-210: p=0.7; miR-320: p=0.8), or shock (miR-16: p=0.5; miR-210: p=0.5; miR-320: p=0.4) were found. Similarly, the concentrations of the microRNAs analyzed did not differ between patients of the different intensive care units as assessed by ANOVA (miR-16: p=0.4; miR-210: p=0.3; miR-320: p=0.08). The concentrations of the microRNAs analysed did not correlate with serum creatinine levels at the start of renal replacement therapy (RRT) (miR-16: r=-0.14 p=0.2; miR-210: r=-0.3 p=0.8; miR-320: r=-0.4, p=0.8).

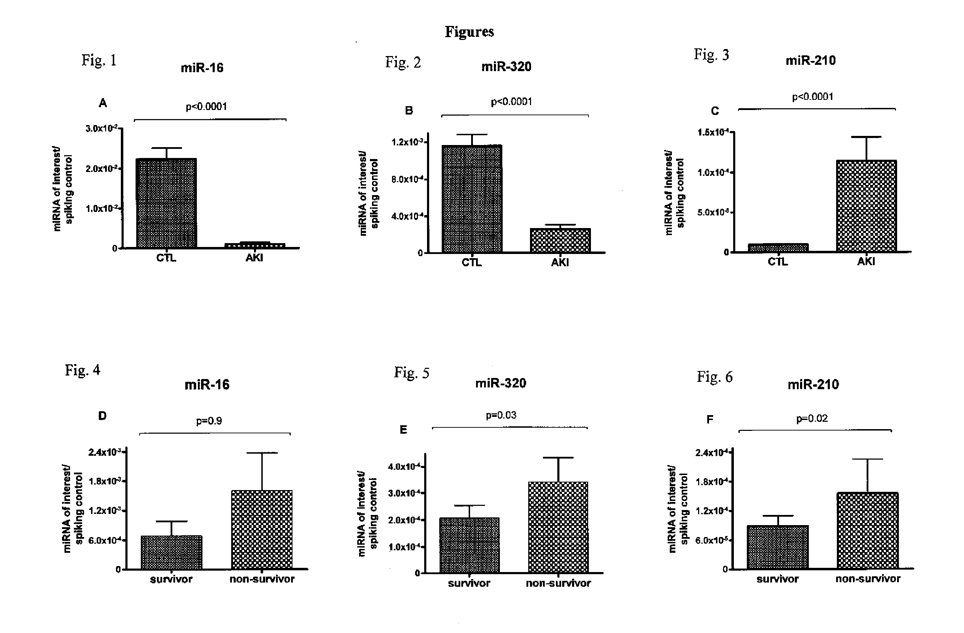

The analytical results for the AKI patients and healthy controls (CTL) of the determined concentration of miR-16 in serum is shown in Figure 1, for miR-320 in Figure 2, and for miR-210 in Figure 3. For the analysis, serum samples were spiked with recombinant miR-54 of Caenorhabditis elegans to 5 fmol/µL as an internal control miR, which was detected in parallel by the quantitative RT-PCR using the TaqMan method with specific reverse transcription primer, amplification primers, and detection oligonucleotide, to normalize for possible differences in the efficiency of the analytical method, including efficiency in RNA isolation from the serum. Spiked serum was used for RNA isolation with the MasterPure ^{(™)} RNA purification kit obtainable from Epicenter Biotechnologies.

Normalization of measurement results for the analyte microRNAs using the detected concentration of the miR-54 (SEQ ID NO 4: UACCCGUAAUCUUCAUAAUCCGAG) was done according to Fichtlscherer et al ("Circulating microRNAs in patients with coronary artery disease", Circ. Res. 677 - 684 (2010)). In short, the concentration of analyte miR, e.g. of miR-210, miR-320, and of miR-16 was calculated from the number of specific amplification products per reaction volume to obtain a calculated initial copy number of each analyte miR per initial sample volume, which initial copy number of analyte miR per initial sample volume was divided by the initial copy number concentration of added control miR, e.g. divided by the calculated concentration of control miR that was obtained from an added concentration of 5 fmol/µL serum, thus accounting for losses during RNA isolation.

Using this normalization of control miR-54 of C. elegans added to 5 fmol/µL initial sample volume, isolation of total RNA, RT-PCR using primer pairs specific for the analyte miR, e.g. for miR-210 and a primer pair specific for the control miR-54, the mean value of the concentration of miR-210 normalized by dividing the calculated initial concentration of miR-210 in the sample by the concentration of added control miR, added to a serum concentration of 5 fmol/µL serum, was determined to a value of 4.00098 x 10⁻⁵. Serum concentrations of miR-210 above a ratio of 4.00098 x 10⁻⁵ (initial concentration of miR-210 calculated from RT-PCR products) / (initial concentration calculated for control miR-54) in patients suffering from AKI indicate a worse prognosis for a period of 28 days, which was the observation period.

Standard curves of the correlation of the concentration of specific RT-PCR amplification products to the original concentration of the respective microRNA in the RNA were pre-determined with at least 4 dilutions of isolated total RNA containing the microRNA at a known concentration, each dilution differing by a factor of 10. Preferably, the known microRNA was added to a known concentration in the serum, and total RNA was isolated from serum, diluted to at least 2 dilution steps, and subjected to RT-PCR using a primer pair specific for the microRNA that was originally added to the serum.

Generally, the analytical method can include the step of measuring the concentration of a known housekeeping microRNA present in serum, and dividing the concentration of the analyte microRNA by the concentration of the housekeeping microRNA for normalization.

The results shown in Figures 1 - 3 demonstrate that in all the AKI patients and control individuals, it could be confirmed that lower than median concentrations of miR-16 and of miR-320 (both p < 0.001), and higher than median concentrations of miR-210 in patients with AKI in comparison to healthy controls were found.

The only significant association that was found were baseline concentrations of miR-16 (r=0.3, p=0.02), miR-210 (r=0.4, pp<0.001), and of miR-320 (r=0.4, pp<0.001) correlating with lactate levels, and baseline concentrations of miR-210 additionally was correlated with heart-rate (r=0.04, p<0.0001) baseline concentrations of miR-320 was correlated with heart-rate (r=0.4, p<0.0001), and noradrenalin dose (r=0.3, p=0.04).

Following the initial analysis, AKI patients underwent renal replacement therapy, and patients were grouped 4 weeks after the therapy in survivors and non-survivors. It was found that generally both these patient groups were comparable with respect to baseline demographics and to the proportion of RIFLE categories as shown in table 1. It was found that the group of survivors and the group of non-survivors only differed with regard to the proportion of patients with metabolic acidosis (p=0.005), and SOFA score (p=0.05). It was found that patients suffering from sepsis have a higher SOFA and higher APACHE II scores, which were deemed to result from more severe cardiovascular and respiratory impairment compared to the non-septic patients.

The analytical results for the AKI patients for survivors and non-survivors are shown in Figure 4 for concentration of miR-16, in Figure 5 for miR-320, and in Figure 6 for the concentration of miR-210. It is found that the concentration of miR-210 (p=0.02) and of miR-320 (p= 0.03) were significantly higher in non-survivors, while miR-16 did not show a difference between non-survivors and survivors.

An initial analysis by the univariate cox proportional hazards analysis was done to determine a possible relationship between circulating concentrations of microRNAs at initiation of RRT and mortality. It was found that in the initial AKI patients, concentrations of miR-16, age, gender, and body mass index were not significantly associated with survival, as shown in the following Table 2.

**Table 2: Univariate and multivariate Cox regression analysis for survival**

| Univariate | | | | Multivariate | | |
|---|---|---|---|---|---|---|
| Variables | HR | 95% CI | p-value | HR | 95% CI | p-value |
| miR-210 (log10) | 1.868 | 1.079 to 3.232 | 0.03* | 1.719 | 0.999 to 2.959 | 0.05* |
| miR-320 (log10) | 2.119 | 1.074 to 4.181 | 0.03* | n.s. | | |
| miR-16 (log10) | 1.299 | 0.753 to 2.242 | 0.3 | | | |
| Age (years) | 0.994 | 0.969 to 1.020 | 0.7 | | | |
| Body mass index (kg/m²) | 0.972 | 0.897 to 1.054 | 0.5 | | | |
| Gender (m/f) | 0.909 | 0.433 to 1.907 | 0.8 | | | |
| Sepsis (yes/no) | 2.257 | 1.071 to 4.753 | 0.03* | n.s. | | |
| Surgery (yes/no) | 2.024 | 0.947 to 4.324 | 0.07 | n.s. | | |
| SOFA score | 1.203 | 1.066 to 1.357 | 0.003* | 1.194 | 1.054 to 1.352 | 0.005* |
| APACHE II score | 1.055 | 1.007 to 1.106 | 0.03* | n.s. | | |

| | | | | | | |
|---|---|---|---|---|---|---|
| CI = confidence interval, HR = hazard ratio, n.s. = non-significant, * = p<0.1 | | | | | | |

From the data of Table 2, it was found that the variables major surgery, sepsis, APACHE II score, and SOFA score as well as concentrations of miR-210, and miR-320 display a prognostic significance at a 10% level. In multivariate analysis, it was found that only the SOFA score (p= 0.005), and miR-210 (p= 0.05) are independent predictors of survival. In ROC curve analysis miR-210 yielded an area under the ROC curve of 0.74 (standard error of the mean: 0.06; 95% confidence interval: 0.53 to 0.78; p= 0.03), while the concentration of miR-320 showed a similar level of significance (area under the ROC curve 0.7; standard error of the mean: 0.07; 95% confidence interval: 0.53 to 0.78; p= 0.03). The concentrations of miR-16 did not reach statistical significance (area under the ROC curve 0.55, standard error of the mean: 0.07, 95% confidence interval: 0.41 to 0.69, p=0.4). For comparison, the area under the curve of the SOFA score gave a value of 0.7 (standard error of the mean: 0.06, 95% confidence interval: 0.57 to 0.82, p=0.005).

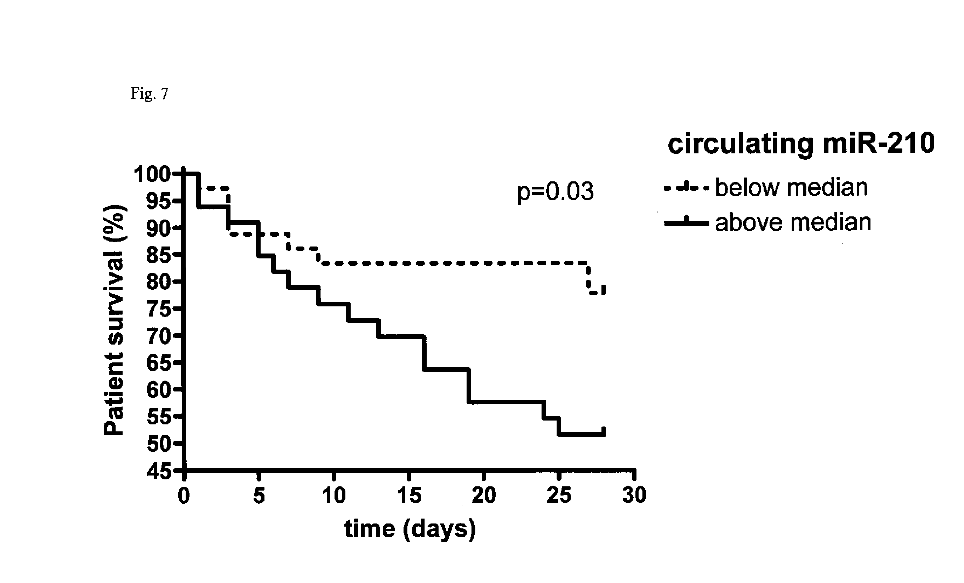

Figure 7 shows the Kaplan-Meier curve of survival at 4 weeks after initiation of RRT, stratified to miR-210 concentration above and below the median concentration value. The Log rank test confirmed the statistical significance for miR-210 (p=0.03), while miR-320 (p=0.08) and miR-16 (p=0.4) did not show statistical significance in this test.

## Claims

1. Method for analysis of kidney status in a sample of body fluid by determining the concentration of an analyte,
**characterized in that** the analyte is miR-210.

2. Method according to claim 1, **characterized by** additionally determining the concentration of at least one of the analytes of the group containing miR-16 and miR-320.

3. Method according to one of the preceding claims, **characterized in that** the step of determining the concentration of an analyte comprises the step of contacting the sample of body fluid with a nucleic acid sequence which comprises a section that is reverse complementary to the analyte, and detecting the concentration of nucleic acid hybrids formed of the nucleic acid sequence comprising the reverse complementary nucleic acid section.

4. Method according to one of the preceding claims, **characterized in that** the nucleic acid sequence containing the nucleic acid section which is reverse complementary to the analyte is immobilized on a carrier substrate.

5. Method according to one of the preceding claims, **characterized in that** the nucleic acid sequence containing the nucleic acid section which is reverse complementary to the analyte is labelled by a detectable chromophore or a combination of a detectable chromophore and a spaced apart quencher to the chromophore.

6. Method according to one of the preceding claims, **characterized in that** the step of determining the concentration of the analyte comprises a reverse transcription reaction using an oligonucleotide as a primer that is specific for the analyte, and a PCR reaction using two oligonucleotides as primers which are specific for the analyte.

7. Method according to claim 6, **characterized in** the amplification product of the PCR reaction is specifically detected by hybridization with a labelled oligonucleotide containing the nucleic acid sequence of the analyte, or a nucleotide sequence that is reverse complementary to the analyte.

8. Method according to one of the preceding claims, **characterized in that** the method comprises the step of
- adding a known control microRNA to a known concentration to the sample,
- isolating total RNA from the sample,
- carrying out RT-PCR on the RNA with specific primer pairs added for each of the analyte and for the control miRNA, respectively,
- calculating the original concentration of analyte by means of the standard curve, which contains the pre-determined correlation of the detected concentration of amplification products of the analyte to the original concentration of miRNA,
- calculating the original concentration of control miRNA by means of a pre-determined standard curve which contains the pre-determined correlation of the detected concentration of amplification products of the control miRNA to the original concentration of control miRNA, and
- forming the quotient of the calculated original concentration of analyte and the calculated concentration of control miRNA.

9. Method according to claim 8, **characterized in that** the quotient, which quotient is indicative of the prognosis for the prospects or risk of acute kidney injury.

10. Method according to one of the preceding claims, **characterized in that** the sample originates from a patient who has not been diagnosed with acute kidney injury.

11. Method according to one of claims 1 to 9, **characterized in that** the sample originates from a patient who has been diagnosed with acute kidney injury.

12. Method according to one of the preceding claims, **characterized by** comparing the detected concentration of the analyte to the concentration of the analyte in healthy persons.

13. Device containing a nucleic acid sequence containing the sequence of miR-210 or a nucleic acid section that is reverse complementary to miR-210 for use as an indicator substance which is specific for kidney status.

14. Device according to claim 13, **characterized by** additionally containing a nucleic acid sequence containing a section that is identical to miR-16 and/or to miR-210, or a nucleotide section which is reverse complementary to miR-16 and/or reverse complementary to miR-210 for use as an indicator substance which is specific for kidney status.

15. Device according to one of claims 13 to 14, **characterized in that** the nucleic acid sequence is immobilized on a carrier substrate.
